# EUROPEAN PATENT SPECIFICATION

(11) **EP 1 911 406 B1**
(45) Date of publication and mention of the grant of the patent: **05.05.2010**
(21) Application number: 06775231.1
(22) Date of filing: 27.07.2006
(51) Int. Cl.: A61B 17/122

(54) **A HEMANGIOMA CLIP WITH A BIOLOGICAL MEMBRANE**
HÄMANGIOM-CLIP MIT BIOLOGISCHER MEMBRAN
CLIP POUR HÉMANGIOME AVEC MEMBRANE BIOLOGIQUE

(30) Priority: 04.08.2005 CN 200510036315
(43) Date of publication of application: 16.04.2008
(73) Proprietor: Summit (GD) Biotech Co., Ltd., Guangdong Guangzhou 510663 (CN)
(72) Inventor: QI, Songtao, Guangzhou, Guangdong 510515 (CN); XU, Guofeng, Guangzhou, Guangdong 510630 (CN)
(74) Representative: Korga, Leokadia
(86) International application number: PCT/CN2006/001878
(87) International publication number: WO 2007/014518

(56) References cited:
- WO-A1-02/32327
- US-A- 4 765 335
- US-A- 5 758 420
- US-B1- 6 251 117

## Description

The present invention relates to a medical device for human implantation, and in particular, to a clip for treating hemangioma.

Hemangiomas, and especially aneurysms, are one of the most common vascular diseases. There are many treatment methods for hemangiomas, but for hemangiomas having certain morphology, applying a clip to the sac at the root (i.e., the site parallel to the vessel wall) so as to close the sac is a simple treatment method which is particularly suitable, especially for sites difficult to expose. This method has been applied in clinical treatment for some time and the short-term efficacy is very satisfactory.

Vascular clips have been employed for firmly clamping aneurisms in blood vessels. It is known to bend the substantially one-piece vascular clips which are made of a resilient round material in such a manner that the two ends of the round material form two branches which press together the clamping-cheeks located at their free ends in a resilient manner. A conventional hemangioma clip is known, for example, from U.S. Patent No. 6,251,117 to Aesculap AG & Co. KG. Such clip has a first and a second branch, which are pivotal resiliently into a closed position whereby, in the region of a cross-over point, the first branch passes through a longitudinal slot in the second branch formed between two parallel webs.

However, because the side of the vascular wall perpendicular to the axis also becomes thinner due to elongation when a hemangioma is formed, closing one side can stretch the other side even thinner and result in a risk of a new hemangioma being formed, thereby lowering treatment efficacy. In addition, a conventional hemangioma clip is not effective for treatment of fusiform aneurisms. In light of the above reasons, there still remains a need for an improved aneurysm clip which overcomes the drawbacks mentioned above.

The present invention aims at providing a new-type of hemangioma clip having a biological membrane, which is relatively hard and covers the vessels located at the site clamped by the clip, so as to prevent further changes of the site, prevent the hemangioma sac from rupturing, and to greatly increase the efficacy of treatment.

Another purpose of the present invention is to provide a method of manufacturing of the hamangioma clip having a biological membrane mentioned above.

In order to attain the above-described aims of the invention, the following technological solution is implemented. The hemangioma clip comprises a metal clip and a biological membrane, wherein the metal clip is made of medical stainless steel or titanium alloy, its configuration includes a first clamping rod and a second clamping rod whose rear parts are connected through a resilient member and whose rear parts cross and overlap, and at the ends of the half collars on the first clamping rod and on the second clamping rod are respectively disposed on the first clamping arm and the second clamping arm. The two ends of the biological membrane are stabilized on the first clamping arm and on the second clamping arm respectively to form a membrane cover.

The biological membrane is made of a diaphragm, the fatty omentum, the pericardium, and the intestinal membranes of pigs, cows or sheep, by crosslinking and fixing, and treating to remove antigens. Such raw materials are easy to obtain, have low cost, and are useful for commercializing and application.

Animal tissues are easy to degrade or to decompose, and conventionally, aldehydes (formaldehyde, glutaraldehyde, and so on) have been adopted to crosslink and fix them, so as to increase stability. However, since aldehydes crosslink with proteins through condensation and the crosslinked product may release toxic aldehydes when degrading, the products fixed with aldehydes have long-term residual toxicity. Such drawbacks may not exist when non-aldehydic fixatives, such as epoxides, diamides, diisocyanates, or carbodiimides, are used. For example, epoxides crosslink with proteins via a ring cleavage reaction and they do not reform after cleaving the ring group. Epoxide-crosslinked tissues degrade among other to alcohols which can be metabolized and do not have the high toxicity of residual aldehydes. Therefore, the treated animal tissues have higher stability when fixed with epoxides than those fixed with aldehydes.

According to modern immunological theory, the antigenicity of animal tissues stems mainly from active groups located at specific sites and in specific conformations, and these active groups include -OH, -NH₂, -SH, etc. The specific conformations result mainly from some specific hydrogen bonding formed by spiral protein chains. The specific sites and conformations are called antigen determinants. When treating the animal ligaments, one or several small, active reagents (e.g., acid anhydrides, acid chlorides, acylamides, epoxides, etc.) which can readily react with these groups are used to bind and block these groups, which in turn effectively minimizes the antigenicity, and in the meantime strong hydrogen bonding reagents (e.g., guanidine compounds) are utilized to form new hydrogen bonds and replace the inherent hydrogen bonding of the specific conformations, which changes the specific conformations and further effectively minimizes the antigenicity.

[0010] After being crosslinked and fixed with an epoxide, the animal membrane tissues are hard to degrade or to decompose. Their antigenecity is eliminated by blocking active groups in protein molecules and changing their conformation. The biological membrane made of such animal tissues has no residual toxicity or chronic immune rejection, while it has good biocompatibility. In addition, the hemangioma clip having a biological membrane may cover the peripheral region of the vessels locating at the site clamped by the clip, and the biological membrane may grow to fuse into the external membrane of the blood vessel, effecting biological reinforcement and leading to ideal efficacy.

In an optimal embodiment, the surface of the biological membrane, made of animal hard-membrane tissues, is an active coating which comprises active components of certain specific polypeptides or glucosaminoglycans to adhere growth actors. One of the polypeptides is produced by 16 lysines (K16), glycine (G), arginine (R), aspartic acid (D), serine (S), proline (P), and cysteine (C), through condensation. The glucosaminoglycan maybe hyaluronic acid, chondroitin sulfate, dermatan sulfate, heparin, heparitin sulfate, or keratan sulfate. These active components facilitate fusing of the vessel wall cells covered by the biological membrane with the vessel wall tissues so as to effect biological reinforcement.

The biological membrane has the same length as the circumference of the collar formed by the respective half collars.

In the method for manufacturing the hemangioma clip having a biological membrane, the biological membrane is prepared with natural animal tissues of hard-membrane as the raw material, according to the following steps:

1) Pretreatment: Initial sterilization is performed using a broad spectrum, highly-effective, low-toxicity bactericide, followed by trimming irregular portions.

2) Fixation: The protein molecules in the membrane raw material are crosslinked and fixed with a fixative.

3) Antigen elimination: The specific active groups of proteins in the raw material, such as -OH, -NH₂, -SH, and so on, are blocked with active reagents, and the specific conformation of thereof is changed, by substituting reagents with strong hydrogen bonds for the special hydrogen bonds in helix chains of protein molecules in the substrate.

Finally, the two ends of the biological membrane obtained through the above steps, are stabilized on the clamping arms of the metal clip, respectively.

As an optimized strategy, the method for preparing the hemangioma clip having a biological membrane further includes an additional step, wherein by means of a coupling agent, the surface of the biological membrane couples with an active coating which contains active components of certain specific polypeptides or glucosaminoglycans so as to adhere growth actors.

The fixative involved in the preparation of the hemangioma clip having a biological membrane may be one or two reagents which readily undergo a crosslinking reaction with a protein molecule, such as, e.g., an epoxide, a diamide, a diisocyanate, or a carbodiimide; epoxides are preferable.

The epoxide may be a monoepoxide of the following formula 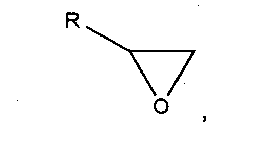 or may be a diepoxide of the following formula 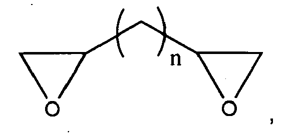 wherein R=CₙH₂ₙ₊₁, and n=0-10.

In the method for preparing the hemangioma clip having a biological membrane, the active reagents may be organic compounds with small molecular weight, such as organic anhydrides, acyl chlorides, acylamides, monocyclic epoxides; and the reagents with strong hydrogen bonds may be guanidine compounds.

In the method for preparing the hemangioma clip having a biological membrane, the coupling agent, which is used for coupling with the active coating containing active components of certain specific polypeptide or glucosaminoglycan may be a diamide, a dianhydride, a diepoxide, or any other reagent with double functional groups to react with groups such as -NH₂, -OH and -COOH.

The advantages of the present invention include: (1) the hemangioma clip having a biological membrane may cover the peripheral region of the vessels located at the site clamped by the clip so as to prevent the hemangioma from deterioration; (2) the hemangioma clip having a good biocompatibility so as to fuse with the external wall of the vessels, exhibiting biological effects of thickening and reinforcing; and (3) the hemangioma having excellent efficacy, safety and reliability for use.

FIG. **1** is a structural view of a hemangioma clip according to one embodiment of the present invention.

FIG. **2** is a magnified view of the region labeled "A" of the hemangioma clip of FIG. **1****.**

Legend: **1 -** First clamping arm; **2 -** Second clamping arm; **3-**Biological membrane; **4 -** Active coating; **5 -** First clamping rod; **6 -** Second clamping rod; **7 -** Resilient member; **8 -** Half collar; **9 -** Half collar; **10 -** Collar.

### EXAMPLES

Example 1

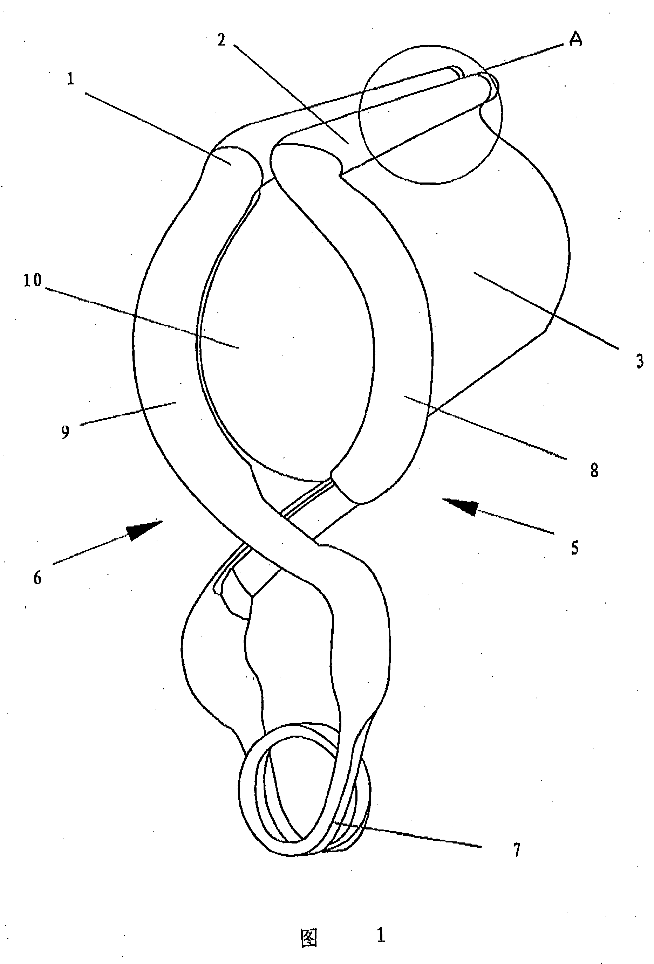

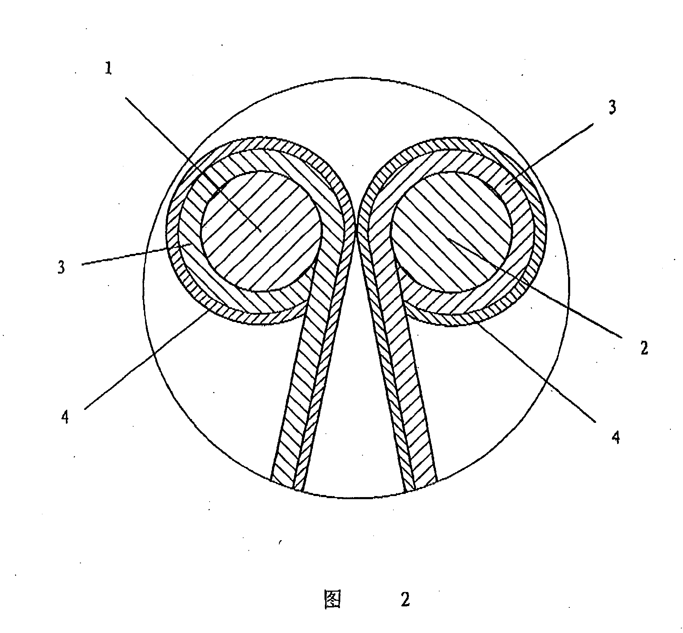

Referring to FIGS. **1** and **2****,** the biological hemangioma clip comprises a metal clip and a biological membrane **3.** The metal clip comprises the first clamping rod **5** and the second clamping rod **6,** whose rear parts are connected through a resilient member **7,** cross and overlap. The first clamping rod **5** and the second clamping rod **6** comprise respectively the half collar **8** on the first clamping rod **5;** and the half collar **9** on the second clamping rod **6,** at whose two ends are disposed the first clamping arm **1** and the second clamping arm **2.**

The two ends of the biological membrane **3** are stabilized on the first clamping arm **1** and the second clamping arm **2,** respectively, to form a membrane cover. The length of the biological membrane **3** is the same as the circumference of the collar **10** formed by the half collar **8** and the half collar **9,** and its breadth is the same as the length of the clamping arm or its two ends are both respectively longer than the clamping arm by about 1-2 mm.

The biological membrane **3** couples with the active coating **4** containing active components such as certain specific polypeptides or glucosaminoglycans to bind growth factors. An exemplary polypeptide is made of 16 lysines (K16), glycine (G), arginine (R), aspartic acid (D), serine (S), proline (P), and cysteine (C), through condensation.

The glucosaminoglycan may be hyaluronic acid, chondroitin sulfate, dermatan sulfate, heparin, heparitin sulfate, or keratan sulfate. The biological membrane is formed from the diaphragm, the fatty omentum, the pericardium and the intestinal membranes from pigs, cows or sheep, by crosslinking and fixing through an epoxide, and treating to remove antigens. The metal clip may be made of medical stainless steel or titanium alloy.

In the method for preparing the hemangioma clip having a biological membrane, the biological membrane is prepared with natural animal tissues of hard-membrane as the raw material, according to the following steps:

1) Pretreatment: Initial sterilization is performed using a broad spectrum bactericide, followed by trimming irregular portions.

2) Fixation: The protein molecules in the membrane raw material are crosslinked and fixed with a fixative.

3) Antigen elimination: The specific active groups of proteins in the raw material, such as -OH, -NH₂, -SH, and so on, are blocked with active reagents, and the specific conformation thereof is changed, by substituting reagents with strong hydrogen bonds for the special hydrogen bonds in helix chains of protein molecules in the substrate.

4) Mediated by a coupling agent, the surface of the biological membrane couples with an active coating which contains active components of certain specific polypeptides or glucosaminoglycans so as to bind growth factors.

Finally, the two ends of the obtained biological membrane are stabilized on the clamping arms of the metal clip with a medical adhesive, respectively. And hence the product of this present invention described herein is made.

The fixative may be one or two of the reagents which readily undergo a crosslinking reaction with a protein molecule, such as an epoxide, a diamide, a diisocyanate, or a carbodiimide. The epoxide may be a monoepoxide of the following formula, 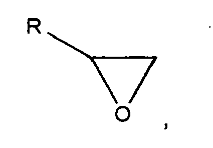 or may be diepoxide of the following formula: 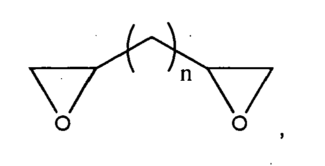 wherein R=CₙH₂ₙ₊₁, and n=0-10.

The active reagents may be organic compounds with small molecular weight, such as organic anhydrides, acyl chlorides, acylamides, monocyclic epoxides; and the reagents forming strong hydrogen bonds may be guanidine compounds.

The coupling agent may be a diamide, a dianhydride, a diepoxide, or any other reagent having double functional groups to undergo a reaction with such groups as -NH₂, -OH and -COOH.

## Claims

1. A hemangioma clip comprising a metal clip made of medical stainless steel or titanium alloy with a first clamping rod (5) and a second clamping rod (6), whose rear parts are connected through a resilient member (7) and cross and overlap, the first clamping rod (5) comprising a first half collar (8) at whose end disposed is a second clamping arm (2), and the second clamping rod (6) comprising a second half collar (9) at whose end disposed is a first clamping arm (1) **characterized in that** the hemangioma clip further comprises a biological membrane (3) stabilized on said first clamping arm (1) and said second clamping arm (2) to form a membrane cover.

2. A method of preparation of biological membrane (3) for the hemangioma clip of claim 1, **characterized in that** said biological membrane is formed from a diaphragm, fatty omentum, pericardium and intestinal membranes of pigs, cows or sheep, by crosslinking and fixing with an epoxide, and treating to remove antigens.

3. The method of preparation of biological membrane (3) for the hemangioma clip of claim 1, **characterized in that** said biological membrane (3) couples with the active coating (4) containing active components such as certain specific polypeptides or glucosaminoglycans so as to bind growth factors.

4. The method of preparation of biological membrane (3) for the hemangioma clip of claim 3, **characterized in that** one of said polypeptides is made of 16 lysines (K16), glycine (G), arginine (R), aspartic acid (D), serine (S), proline (P), and cysteine (C), through condensation, and said glucosaminoglycan may be hyaluronic acid, chondroitin sulfate, dermatan sulfate, heparin, heparitin sulfate, or keratan sulfate.

5. The method of preparation of biological membrane (3) for the hemangioma clip of any of claims 2-4 **characterized in that** the length of said biological membrane (3) is the same as the circumference of a collar (10) formed by a fist half collar (8) and a second half collar (9).

6. The method of preparation of biological membrane (3) for the hemangioma clip of claim 2, **characterized in that** said biological membrane (3) is prepared from hard-membrane natural animal tissues taken as the raw material, according to the following steps:
1) Pretreatment: Initial sterilization is performed using a broad spectrum, highly-effective, low-toxicity bactericide, followed by trimming irregular portions;
2) Fixation: The protein molecules in the biological membrane (3) are crosslinked and fixed with a fixative;
3) Antigen elimination: The specific active groups of proteins in the biological membrane (3), such as -OH, -NH₂, -SH, and so on, are blocked with active reagents, and the specific conformation of them is changed, by substituting reagents forming strong hydrogen bonds for the special hydrogen bonds in helix chains of protein molecules in the substrate; finally, the two ends of the obtained biological membrane (3) are stabilized on the clamping arms of the metal clip with a medical adhesive, respectively.

7. The method of preparation of biological membrane (3) for the hemangioma clip of claim 6 **characterized in that** the method comprises further an additional step, wherein by means of a coupling agent, the surface of the biological membrane (3) couples with the active coating (4) which contains active components of certain specific polypeptides or glucosaminoglycans so as to bind growth actors.

8. The method of preparation of biological membrane (3) for the hemangioma clip of claim 7? **characterized in that** one of said polypeptides is made of 16 lysines (K16), a glycine (G), an arginine (R), an aspartic acid (D), a serine (S), a proline (P), and a cysteine (C) through condensation, and the glucosaminoglycan may be hyaluronic acid, chondroitin sulfate, dermatan sulfate, heparin, heparitin sulfate, or keratan sulfate.

9. The method of preparation of biological membrane (3) for the hemangioma clip of claims 5 or 6, **characterized in that** said fixative may be one or two of the reagents readily undergoing a crosslinking reaction with a protein molecule, such as an epoxide, a diamide, a diisocyanate, or a carbodiimide; and said epoxide may be a monoepoxide of the following formula 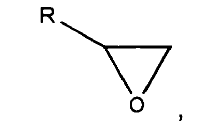 or a diepoxide of the following formula 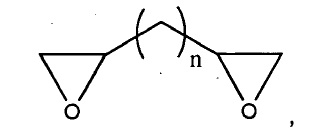 R=CₙH₂ₙ₊₁, and n=0-10.

10. The method of preparation of biological membrane (3) for the hemangioma clip of claims 5 or 6, **characterized in that** said active reagent is an organic acid, an anhydride with a low molecular weight, an acetyl chloride, an acrylamide, or a monocyclic oxide, and said reagent forming strong hydrogen bonds is a guanidine compound.

11. The method of preparation of biological membrane (3) for the hemangioma clip of claim 6, **characterized in that** the coupling agent is an acetyl diamide, a dianhydride, a diepoxide, or any other compound with double functional groups, which participates in a condensation reaction with groups, such as -NH₂, -OH, or -COOH.

## Patentansprüche

1. Hämangiom-Klemme, welche eine aus medizinischem rostfreiem Edelstahl oder aus einer Titanlegierung hergestellte Metallklemme mit einer ersten Klemmstange (5) und einer zweiten Klemmstange (6) aufweist, deren hintere Teile durch ein elastisches Element (7) verbunden sind und sich überkreuzen und überlappen, wobei die erste Klemmstange (5) eine erste Halbmanschette (8) aufweist, an deren Ende ein zweiter Klemmarm (2) angeordnet ist, und wobei die zweite Klemmstange (6) eine zweite Halbmanschette (9) aufweist, an deren Ende ein erster Klemmarm (1) angeordnet ist, **dadurch gekennzeichnet, dass** die Hämangiom-Klemme des Weiteren eine biologische Membran (3) aufweist, welche auf dem ersten Klemmarm (1) und auf dem zweiten Klemmarm (2) stabilisiert ist, um eine Membranabdeckung zu bilden.

2. Verfahren der Herstellung einer biologischen Membran (3) für die Hämangiom-Klemme nach Anspruch 1, **dadurch gekennzeichnet, dass** die biologische Membran aus einem Diaphragma, einem fettreichen Omentum, einem Perikard und Darmmembranen von Schweinen, Kühen oder Schafen durch Vernetzung und Fixierung mit einem Epoxid und einer Behandlung zur Entfernung von Antigenen gebildet ist.

3. Verfahren der Herstellung einer biologischen Membran (3) für die Hämangiom-Klemme nach Anspruch 1, **dadurch gekennzeichnet, dass** die biologische Membran (3) mit der aktiven Beschichtung (4) gekoppelt ist, welche aktive Bestandteile wie bestimmte spezifische Polypeptide oder Glukosaminoglykane enthält, um Wachstumsfaktoren zu binden.

4. Verfahren der Herstellung einer biologischen Membran (3) für die Hämangiom-Klemme nach Anspruch 3, **dadurch gekennzeichnet, dass** eines der Polypeptide durch Konsensation aus 16 Lysinen (K16), Glycin (G), Arginin (R), Asparaginsäure (D), Serin (D), Prolin (P) und Cystein (C) hergestellt ist und dass es sich bei dem Glukosaminoglykan um Hyaluronsäure, Chondroitinsulfat, Dermatansulfat, Heparin, Heparinsulfat oder Keratansulfat handeln kann.

5. Verfahren der Herstellung einer biologischen Membran (3) für die Hämangiom-Klemme nach einem der Ansprüche 2-4, **dadurch gekennzeichnet, dass** die Länge der biologischen Membran (3) gleich ist wie der Umfang einer Manschette (10), die von einer ersten Halbmanschette (8) und einer zweiten Halbmanschette (9) gebildet ist.

6. Verfahren der Herstellung einer biologischen Membran (3) für die Hämangiom-Klemme nach Anspruch 2, **dadurch gekennzeichnet, dass** die biologische Membran (3) gemäß folgenden Schritten aus natürlichen tierischen Hartmembrangeweben hergestellt ist, welche als Rohmaterial herangezogen werden:
1) Vorbehandlung: Unter Verwendung eines hoch wirksamen Breitspektrum-Bakterizids mit niedriger Toxizität wird eine Eingangssterilisation durchgeführt, gefolgt vom Zurechtstutzen unregelmäßiger Anteile;
2) Fixierung: Die Proteinmoleküle in der biologischen Membran (3) werden vernetzt und mit einem Fixierungsmittel fixiert;
3) Antigenelimination: Die spezifischen aktiven Gruppen von Proteinen in der biologischen Membran (3), wie beispielsweise -OH, -NH₂, -SH und so weiter, werden mit aktiven Reagenzien blockiert, und ihre spezifische Konformation wird geändert, indem die speziellen Wasserstoffbrückenbindungen in Helixketten von Proteinmolekülen in dem Substrat durch Reagenzien, welche starke Wasserstoffbrückenbindungen bilden, ersetzt werden; schließlich werden die beiden Enden der erhaltenen biologischen Membran (3) jeweils mit einem medizinischen Kleber auf den Klemmarmen der Metallklemme stabilisiert.

7. Verfahren der Herstellung einer biologischen Membran (3) für die Hämangiom-Klemme nach Anspruch 6, **dadurch gekennzeichnet, dass** das Verfahren einen zusätzlichen Schritt umfasst, bei dem die Oberfläche der biologischen Membran (3) mithilfe eines Kopplungsmittels an die aktive Beschichtung (4) gekoppelt wird, die aktive Bestandteile bestimmter spezifischer Polypeptide oder Glukosaminoglykane enthält, um Wachstumsfaktoren zu binden.

8. Verfahren der Herstellung einer biologischen Membran (3) für die Hämangiom-Klemme nach Anspruch 7, **dadurch gekennzeichnet, dass** eines der Polypeptide durch Konsensation aus 16 Lysinen (K16), Glycin (G), Arginin (R), Asparaginsäure (D), Serin (D), Prolin (P) und Cystein (C) hergestellt ist und dass es sich bei dem Glukosaminoglykan um Hyaluronsäure, Chondroitinsulfat, Dermatansulfat, Heparin, Heparinsulfat oder Keratansulfat handeln kann.

9. Verfahren der Herstellung einer biologischen Membran (3) für die Hämangiom-Klemme nach Anspruch 5 oder 6, **dadurch gekennzeichnet, dass** es sich bei dem Fixierungsmittel um eines oder zwei der Reagenzien handelt, die leicht eine Vernetzungsreaktion mit einem Proteinmolekül eingehen, wie beispielsweise ein Epoxid, ein Diamin, ein Diisocyanat oder ein Carbodiimid, und wobei es sich bei dem Epoxid um ein Monoepoxid der folgenden Formel 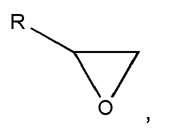 oder um ein Diepoxid der folgenden Formel 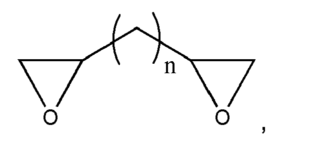 mit R=CₙH₂ₙ₊₁ und n=0-10, handeln kann.

10. Verfahren der Herstellung einer biologischen Membran (3) für die Hämangiom-Klemme nach Anspruch 5 oder 6, **dadurch gekennzeichnet, dass** es sich bei dem aktiven Reagenz um eine organische Säure, ein Anhydrid mit niedrigem Molekulargewicht, ein Acetylchlorid, ein Acrylamid oder ein monozyklisches Oxid handelt, und wobei es sich bei dem Reagenz, welches starke Wasserstoffbrückenbindungen bildet, um eine Guanidinverbindung handelt.

11. Verfahren der Herstellung einer biologischen Membran (3) für die Hämangiom-Klemme nach Anspruch 6, **dadurch gekennzeichnet, dass** es sich bei dem Kopplungsmittel um ein Acetyldiamid, ein Dianhydrid, ein Diepoxid oder eine beliebige andere Verbindung mit doppelten funktionellen Gruppen handelt, das an einer Kondensationsreaktion mit Gruppen wie beispielsweise -NH₂, -OH oder -COOH teilnimmt.

## Revendications

1. Clip pour hémangiome comprenant un clip en métal en acier inoxydable médical ou alliage de titane médical avec une première barre de serrage (5) et une seconde barre de serrage (6), dont les parties arrières sont connectées par un élément souple (7) et se croisent et se chevauchent, la première barre de serrage (5) comprenant un premier demi-collier (8) à l'extrémité duquel est disposé un deuxième bras de serrage (2), et la seconde barre de serrage (6) comprenant un second demi-collier (9) à l'extrémité duquel est disposé un premier bras de serrage (1) **caractérisé en ce que** le clip pour hémangiome comprend en outre une membrane biologique (3) stabilisée sur ledit premier bras de serrage (1) et ledit second bras de serrage (2) pour former une membrane de couverture.

2. Procédé de préparation d'une membrane biologique (3) pour le clip pour hémangiome selon la revendication 1, **caractérisé en ce que** ladite membrane biologique est formée à partir d'un diaphragme, d'un épiploon gras, d'un péricarde et de membranes intestinales de porcs, de vaches ou de moutons, par réticulation et fixation avec un époxyde, et traitement pour éliminer les antigènes.

3. Procédé de préparation d'une membrane biologique (3) pour le clip pour hémangiome selon la revendication 1, **caractérisé en ce que** ladite membrane biologique (3) se couple avec le revêtement actif (4) contenant des composants actifs tels que certains polypeptides spécifiques ou glucosaminoglycanes afin de lier les facteurs de croissance.

4. Procédé de préparation d'une membrane biologique (3) pour le clip pour hémangiome selon la revendication 3, **caractérisé en ce que** l'un desdits polypeptides est constitués de 16 lysines (K16), de glycine (G), d'arginine (R), d'acide aspartique (D), de sérine (S), de proline (P), et de cystéine (C), par condensation, et ledit glucosaminoglycane peut être l'acide hyaluronique, le sulfate de chondroïtine, le sulfate de dermatane, l'héparine, le sulfate d'héparitine, ou le sulfate de kératan.

5. Procédé de préparation d'une membrane biologique (3) pour le clip pour hémangiome selon l'une quelconque des revendications 2 à 4, **caractérisé en ce que** la longueur de ladite membrane biologique (3) est la même que la circonférence d'un collier (10) formé par un premier demi-collier (8) et un second demi-collier (9).

6. Procédé de préparation d'une membrane biologique (3) pour le clip pour hémangiome selon la revendication 2, **caractérisé en ce que** ladite membrane biologique (3) est préparée à partir de tissus animaux naturels de membrane dure prélevés en tant que matière première, selon les étapes suivantes :
1) prétraitement : la stérilisation initiale est réalisée en utilisant un bactéricide à large spectre, très efficace et peu toxique, suivi par la découpe des parties irrégulières ;
2) fixation : les molécules protéiques dans la membrane biologique (3) sont réticulées et fixées avec un fixateur ;
3) élimination des antigènes : les groupes actifs spécifiques de protéines dans la membrane biologique (3), tels que -OH, -NH2, -SH, et ainsi de suite, sont bloqués avec des réactifs actifs, et leur conformation spécifique est modifiée, par substitution de réactifs formant des liaisons hydrogène fortes pour les liaisons hydrogène spéciales dans les chaînes d'hélice de molécules protéiques dans le substrat ; finalement, les deux extrémités de la membrane biologique obtenue (3) sont stabilisées sur les bras de serrage du clip en métal avec un adhésif médical, respectivement.

7. Procédé de préparation d'une membrane biologique (3) pour la pince pour hémangiome selon la revendication 6, **caractérisé en ce que** le procédé comprend en outre une étape supplémentaire, dans lequel par les moyens d'un agent de couplage, la surface de la membrane biologique (3) se couple avec le revêtement actif (4) qui contient des composants actifs de certains polypeptides spécifiques ou glucosaminoglycanes afin de lier les facteurs de croissance.

8. Procédé de préparation d'une membrane biologique (3) pour le clip pour hémangiome selon la revendication 7, **caractérisé en ce que** l'un desdits polypeptides est constitués de 16 lysines (K16), d'une glycine (G), d'une arginine (R), d'un acide aspartique (D), d'une sérine (S), d'une proline (P), et d'une cystéine (C) par condensation, et le glucosaminoglycane peut être l'acide hyaluronique, le sulfate de chondroïtine, le sulfate de dermatane, l'héparine, le sulfate d'héparitine, ou le sulfate de kératan.

9. Procédé de préparation d'une membrane biologique (3) pour le clip pour hémangiome selon les revendications 5 ou 6, **caractérisé en ce que** ledit fixateur peut être un ou deux des réactifs subissant facilement une réaction de réticulation avec une molécule protéique, telle qu'un époxyde, un diamide, un diisocyanate, ou un carbodiimide ; et ledit époxyde peut être un mono-époxyde de la formule suivante 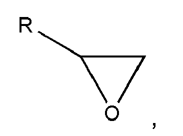 ou un diépoxyde de la formule suivante 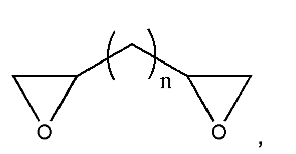 R = CₙH₂ₙ₊₁, et n = 0 à 10.

10. Procédé de préparation d'une membrane biologique (3) pour le clip pour hémangiome selon les revendications 5 ou 6, **caractérisé en ce que** ledit réactif actif est un acide organique, un anhydride avec une faible masse moléculaire, un chlorure d'acétyle, un acrylamide, ou un oxyde monocyclique, et ledit réactif formant de fortes liaisons hydrogène est un composé guanidine.

11. Procédé de préparation d'une membrane biologique (3) pour le clip pour hémangiome selon la revendication 6, **caractérisé en ce que** l'agent de couplage est un acétyl diamide, un dianhydride, un diépoxyde, ou tout autre composé ayant deux groupes fonctionnels, qui prend part à une réaction de condensation avec des groupes tels que -NH₂, -OH, ou -COOH.
